# EUROPEAN PATENT SPECIFICATION

(11) **EP 0 783 275 B1**
(45) Date of publication and mention of the grant of the patent: **12.12.2001**
(21) Application number: 95931654.8
(22) Date of filing: 14.09.1995
(51) Int. Cl.: A61B 17/56, A61B 17/74, A61B 17/58, A61B 17/16, A61F 2/46

(54) **INSTRUMENTATION FOR PROXIMAL FEMORAL COMPACTION BROACHING**
ZUFÜHRWERKZEUG ZUR VERDICHTETEN RÄUMUNG DES PROXIMALEN FEMORALSCHAFTES
INSTRUMENTS POUR POSE DE BROCHES PAR COMPACTAGE DE L'EXTREMITE SUPERIEURE DU CANAL FEMORAL

(30) Priority: 21.09.1994 US 309592
(43) Date of publication of application: 16.07.1997
(73) Proprietor: Johnson, Lanny L., East Lansing, Michigan 48823 (US)
(72) Inventor: Johnson, Lanny L., East Lansing, Michigan 48823 (US)
(74) Representative: Haffner, Thomas M., Dr.
(86) International application number: US9511055
(87) International publication number: WO9609011

(56) References cited:
- WO-A-93/01773
- DE-C- 3 630 069
- US-A- 1 384 330
- US-A- 4 524 766
- US-A- 4 657 002
- US-A- 5 057 112
- US-A- 5 100 407
- US-A- 5 437 675

## Description

### BACKGROUND OF THE INVENTION

The present invention relates to implanting prosthesis devices, and, more particularly, to the preparation of the femur for implanting a prosthetic hip replacement device therein.

Bone disease and injuries to joints often make it necessary or desirable to replace a natural joint with an artificial prosthesis. One such replacement involves the fixation of a prosthetic hip joint to a proximal end of the femur. The femur contains a hollow intramedullary femoral canal running through a central axis thereof. When implanting the joint prosthesis into the femur, it is desirable to fix the prosthesis to the femur in such a manner that the stem of the prosthesis lies along the central axis of the femur. In preparing the proximal end of the femur to receive the prosthesis, the femoral head is first removed, exposing the femoral canal. If the femoral canal is large enough to accommodate the prosthesis, the prosthesis is then inserted into the canal and fixed to the femur either by an interference fit or by cement fixation. In certain circumstances, the femoral canal is not wide enough to accommodate the prosthesis; thus, the canal is enlarged by drilling or the like to a size appropriate for receiving the stem of the prosthesis at the proximal end of the femur. The femur comprises hard cortical bone surrounding softer porous bone, which lines the femoral canal. Prior to and after drilling to open the femoral canal for insertion of the prosthesis therein, the density of the porous bone remains low at the wall of the femoral canal. Due to the low-density canal wall, fixation of the prosthesis to the femur may deteriorate over time.

A prior art device for preparing the cavity of a bone for implanting a prosthetic hip replacement device therein is disclosed in WO 93/01773. The apparatus according to WO 93/01773 is utilized for performing hip prosthesis revision surgery including preparation of the cavity left after removal of the original prosthesis. A tamp having longitudinal passageway extending longitudinally through the stem portion thereof and a guidewire positioned in the cavity function to compact bone graft material in the cavity and form a precisely contoured hip prosthesis receiving cavity.

A compactor instrument as defined in the preamble of claim 1 is disclosed in DE-A-3 630 069.

### SUMMARY OF THE INVENTION

An object of the present invention is to provide a series of instruments for use in preparing an internal wall of a cavity in bone for implantation of a prosthesis device. The instruments are sized and configured for sequential dilation of the bone lining the canal. The dilation results in compacting bone, thereby defining high density canal walls, which increases the fixation properties between the bone and prosthesis. In accordance with the principles of the present invention, this objective is obtained by providing a kit of instruments for preparing an internal wall of a cavity in bone for implantation of a prosthesis therein, the instrumentation kit comprising a first compactor instrument having a proximal portion and a distal portion, said distal portion being configured and sized relative to the dimensions of said cavity so as to compact bone on said wall when the distal portion of the first instrument is inserted within the cavity. The instrumentation kit further comprises at least one additional compactor instrument having a proximal portion and a distal portion, said distal portion of each additional compactor instrument being configured and sized such that after compaction of bone on the wall by at least said first instrument the wall of the cavity is further compacted when the distal portion of each additional compactor instrument is inserted within the cavity.

The instrumentation kit is particularly appropriate for preparing a femur for implantation of a prosthesis therein, the femur including a femoral shaft having an exposed femoral canal therein defined by sidewalls, the prosthesis being constructed and arranged to be received within the femoral canal. The kit includes at least a first compactor instrument having a proximal portion and a distal broach portion, the broach portion being constructed and arranged to conform substantially to a proximal end of the femoral canal so as to compact bone in the sidewalls thereof without intentionally removing bone from the femoral canal. At least one second compactor instrument is provided which also has a proximal portion and a distal broach portion, the latter being constructed and arranged to conform substantially to a shape of the prosthesis so as to further compact bone in the sidewalls of the proximal end of the femoral canal so that they conform substantially to the shape of the prosthesis. At least one third compactor instrument is provided and has a proximal portion and an elongated distal broach portion. The elongated broach portion is constructed and arranged to extend into the femoral canal and compact bone in sidewalls of the femoral canal at a location distal to previously compacted sidewalls of the proximal end of the femoral canal.

With the invention an instrumentation kit is provided for preparing internal walls of a cavity in bone for receiving a medical device within the cavity. The preparation includes the steps of selecting a sizing instrument capable of compacting, which comprises the internal wall of the cavity; compacting the bone which comprises the internal wall, without intentionally removing bone from the cavity, by inserting the selected sizing instrument within the cavity; and continuing compacting the bone until the sidewalls of the cavity include dense, compacted bone resisting migration or translocation of a medical device placed therein.

Other objects, features and characteristics of the present invention, as well as the function of the related elements of the structure, and the economics of manufacture, will become more apparent upon consideration of the following detailed description and appended claims with reference to the accompanying drawings, all of which form a part of the specification.

### BRIEF DESCRIPTION OF THE DRAWINGS

FIG. 1 is a perspective view of a first femoral compactor instrument for use in compacting bone in the femur, provided in accordance with the principles of the present invention;
FIG. 2 is a perspective view of a second type of femoral compactor instrument constructed and arranged to conform to the configuration of a prosthesis to be inserted into the femur;
FIG. 3 is a perspective view of a third or final type of femoral compactor instrument for compacting bone deep within the femoral shaft;
FIG. 4 is a partial schematic view of the compactor instrument of FIG. 1, shown inserted into the femoral canal and compacting bone at a proximal end of the femur;
FIG. 5 is a view of the compactor instrument of FIG. 2, shown in position to further compact bone at the proximal end of the femur; and
FIG. 6 is a view of the compactor instrument of FIG. 3 shown in position to compact bone within the femoral shaft.

### DETAILED DESCRIPTION OF THE PRESENTLY PREFERRED EXEMPLARY EMBODIMENT

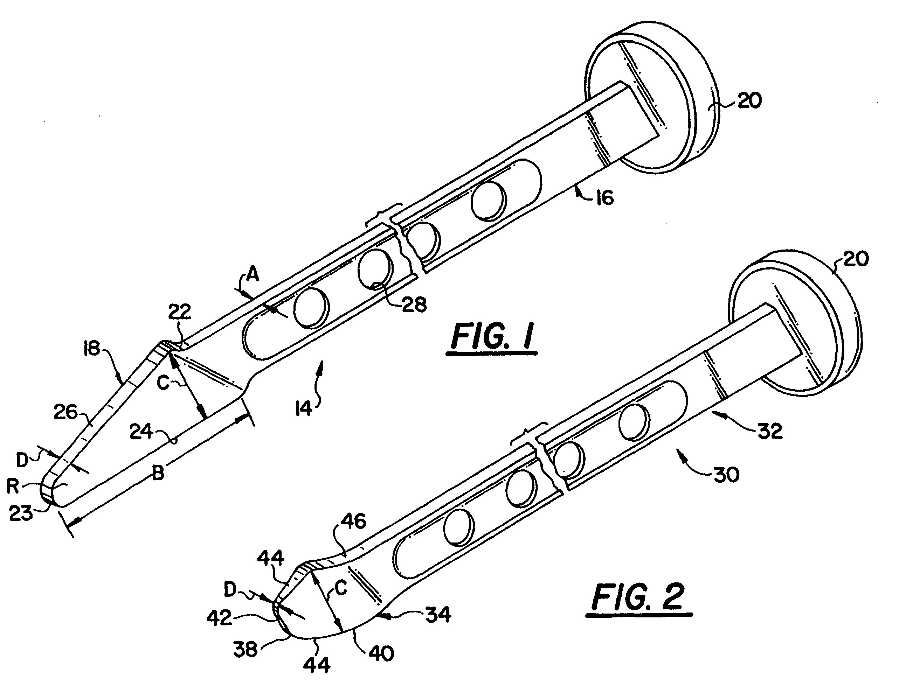

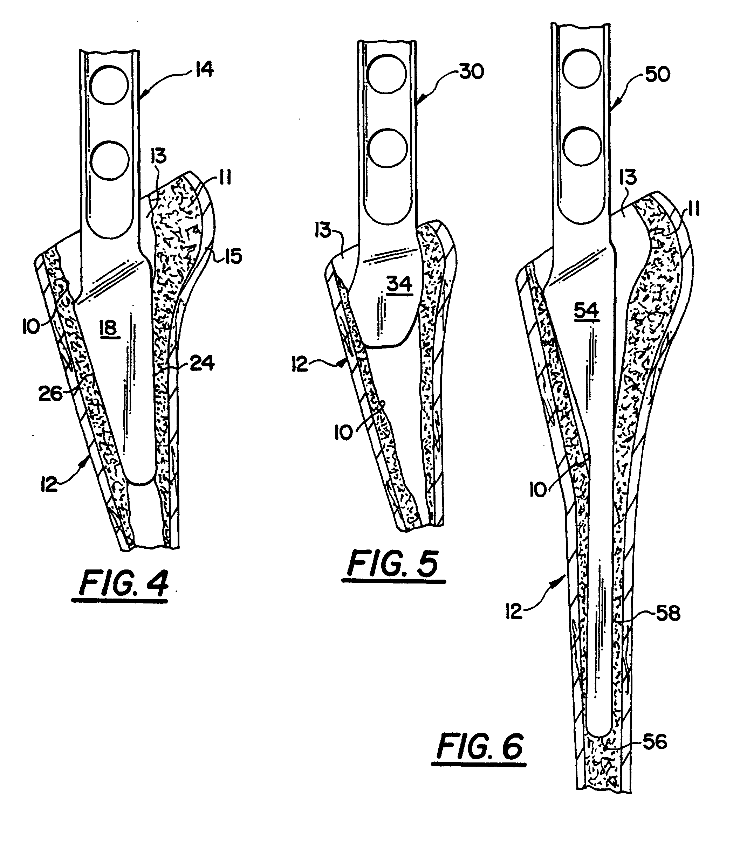

FIGS. 1-3 show a series of femoral compaction instruments defining a kit for use in preparing a femoral cavity or canal 10 (FIGS. 4-6) of a femur 12 for insertion of a femoral prosthesis device (not shown) into the canal 10.

FIG. 1 illustrates a first femoral compactor instrument, generally indicated at 14, comprising a proximal portion 16 and a distal broach portion 18. The proximal portion 16 terminates in an enlarged head 20 which is constructed and arranged to be contacted by a hammer or mallet for use in inserting the instrument 14 into the femur, as will be explained below. The proximal portion 16 is of generally rectangular cross-section having a width A of approximately 10 mm. The proximal portion 16 merges with the broach portion 18 via curved segment 22. The broach portion 18 is constructed and arranged to conform substantially to a proximal end of the femoral canal 10. Thus, as shown in FIG. 1, the broach portion 18 is of generally triangular configuration having a blunt, rounded tip 23. The radius R of the tip 23 is approximately 5 mm. The broach portion 18 has a planar base segment 24, approximately 70 mm in length, as shown by dimension B. The base segment 24 merges smoothly with the rounded tip 23 at one end thereof and merges smoothly with segment 22 at the other end thereof. The broach portion 18 also includes an upper segment 26, which extends from the tip 23 towards the curved segment 22 at an acute angle with respect to the base segment 24. The maximum height C of the broach portion 18 is approximately 25 mm, and the width D is in the range of 2-4 mm. It is within the contemplation of the invention to include a plurality of initial instruments 14 with the kit, each instrument 14 having slightly increasing broach portion widths. For example, one instrument 14 may have a broach portion width D of 2 mm, another, 3 mm and yet another, 4 mm. The instrument 14 is used to compact bone in the femoral canal 10, and the availability of a series of increasing widths for the broach portion 18 assists in achieving the desired dilation of the femoral canal 10, as will be explained more fully below.

The broach portion 18 is formed integrally with the proximal portion 16, preferably from stainless steel or like material suitable for medical purposes. As shown in FIG. 1, the broach portion 18 is solid, while in the illustrated embodiment, the proximal portion 16 includes a plurality of spaced bores 28 therethrough which reduces the amount of material required to manufacture the instrument 14, without diminishing its strength. Of course, the proximal portion 16 may be formed as a solid member, if desired.

FIG. 2 shows a second type of femoral compactor instrument, generally indicated at 30, including a proximal portion 32 and a distal broach portion 34. The proximal first initial instrument 14 of FIG. 1. Thus, the description of the proximal portion 32 need not be repeated here. The broach portion 34 is a solid member constructed and arranged to conform generally to the configuration of the prosthesis to be implanted into the femoral canal 10. In the illustrated embodiment, broach portion 34 is generally short and blunt, as compared to the corresponding portion of instrument 14 of FIG. 1, so as to permit bone in a different portion of the femoral canal 10 to be compacted. The broach portion 34 has a maximum height C of approximately 25 mm and a width D which tapers from approximately 10 mm at the juncture between the proximal portion 32 and the broach portion 34 to approximately 5 mm at the tip 38 of the broach portion 34. Portion 34 includes a curved segment 40, an end segment 42, two tapered segments 44, each extending from the end segment 42, and an upper curved segment 46 merging with the proximal portion 32. These peripheral segments of the broach portion 34 are constructed and arranged to compact bone in the femoral canal 10. It is contemplated by the invention to use instrument 30, after instrument 14 has been employed, to further compact bone in preparing the femur for receiving the prosthesis, as will become apparent below. Further, it is contemplated to include a plurality of these second type of instruments 30 with the kit, each such instrument 30 having slightly increasing broach portion widths.

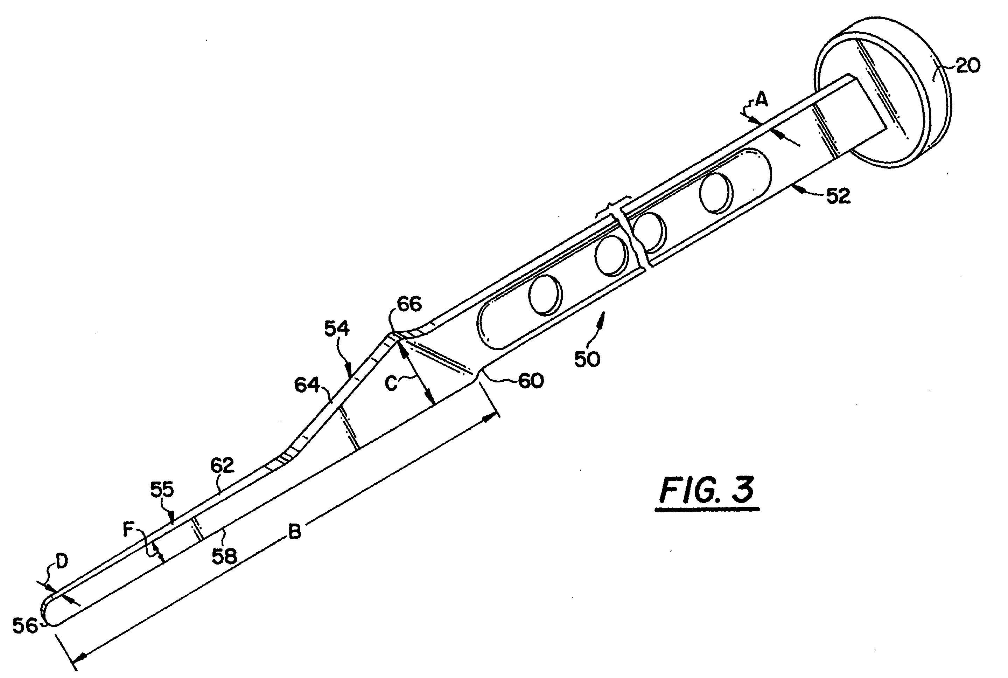

FIG. 3 shows a third, or final type of femoral compactor instrument contained in the kit. The third femoral compactor, generally indicated at 50, is used preferably after compaction has been performed with the second type of instrument 30. As with the instrument of FIG. 2, the proximal portion 52 of instrument 50 is identical to the proximal portion 16 of the instrument of FIG. 1. As shown in FIG. 3, the distal broach portion 54 of instrument 50 is similar to the broach portion 18 of instrument 14, but further includes an elongation, generally indicated at 55, to the distal broach portion 54. The broach portion 54 of instrument 50 has an overall length B of approximately 160 mm and a maximum height C of approximately 25 mm. The elongation 55 has a length of approximately 90 mm, a height F of approximately 9 mm, and a width D which tapers from approximately 10 mm at one end thereof to approximately 5 mm at the distal end 56 of the broach portion 54.

As shown in FIG. 3, the instrument 50 includes a planar base segment 58 spanning the length of the broach portion 54. The base segment 58 merges smoothly with the rounded tip 56 and also merges smoothly with the proximal portion S2 at end 60 thereof. The broach portion 54 also includes an upper segment 62, which extends generally parallel to the base segment 58 from the tip 56 towards the proximal portion 52, a distance of approximately 90 mm. A top segment 64 extends at an acute angle from the upper segment 62 and merges with the proximal portion 52 at end 66. These peripheral segments are constructed and arranged to compact bone deep within the femoral canal 10. The broach portion 54 is solid and made of stainless steel or similar materials. As with the other types of instruments in the complete kit, it is contemplated to include a plurality of the third or final type, such instruments 50 having slightly increasing broach portion widths.

With reference to FIGS. 4-6, the use of the instruments of the kit to prepare a femur for receiving a prosthesis will be described. The first step in a procedure for preparing the femur is to expose the proximal femoral neck and shaft of the femur by removing the femoral head (not shown) . The orientation and direction of the femoral canal 10 is then determined for proper instrument placement. If the canal width is narrow, or a cortical fit is desired, the surgeon first drills the canal 10 in a reverse drill operating mode to prevent removal of bone from the canal 10. If such a drilling operation is not possible, then a forward aggressive drilling mode is used, alternated with reverse drilling, so as to preserve bone fragments within the canal for compaction along the anticipated prosthesis route. If the canal width is larger than the prosthesis to be used, then no drilling is required, and the instruments comprising the invention are sufficient to prepare the femur for the prosthesis placement.

When the instruments contained in the kit are utilized to compact bone to prepare the femur for the prosthesis implant, a 2 mm wide initial femoral compactor 14 first is grasped at the proximal portion 16 thereof and is pushed and/or impacted with a mallet on its head 20 to insert the broach portion 18 thereof into the proximal portion 13 of the femoral canal 10, such that the peripheral segments of the broach portion 18, in particular, segments 24 and 26, displace the softer bone 11 in the sidewalls of the canal 10. The instrument 14 is then removed from the canal 10. After compaction with the 2 mm width initial instrument 14, the 3 mm and the 4 mm width instruments 14 may be inserted sequentially into the canal 10 to further compact bone in the sidewalls thereof, if desired.

Next, as shown in FIG. 5, the second type of femoral compactor instrument 30 is inserted into the femoral cavity 10 in a similar manner as the initial compactor instrument(s) 14, and along the same orientation plane. Instrument 30 is also pushed and/or impacted at its head 20 so the peripheral surfaces of broach portion 34, particularly segments 40 and 44, compact bone in the sidewalls of the canal 10 so that the compacted sidewalls correspond to the shape of the prosthesis to be inserted into the canal 10. It is within the contemplation of the invention to customize the geometry of the broach portion 34 of the second type of femoral compactor instrument 30 to correspond to the geometry of the implant under consideration. Additional instruments 30 having slightly increasing broach portion widths can be used sequentially to dilate a portion of the canal 10, if desired.

Next, the final type of femoral compactor instrument 50 is inserted into the canal 10 in the manner described above, along the same orientation plane as the previously employed instruments. The elongated broach portion 54 of instrument 50 creates compaction of the softer bone 11 in the sidewalls deep within the femoral canal at a location distal to the previously compacted proximal portion 13 of the femoral canal 10, as shown in FIG. 6. If desired, additional instruments 50 having slightly increasing widths can be used sequentially to dilate a portion of the canal 10 deep within the femur.
After using the instruments of the kit to compact bone of the femur, the femur is now ready to receive a prosthesis (not shown) therein. The procedure which has been described for preparing the femur may be used when implanting either a cemented or non-cemented prosthesis.

The method described for preparing a femur for insertion of a femoral component for total hip replacement provides an effective means of compacting bone by the use of a series of compaction instruments to produce dense sidewalls of the canal 10 for receiving the prosthesis. Such density of the sidewalls of the canal 10 ensures a degree of bone compactness which creates high resistance to migration or translocation of the prosthesis inserted into the prepared femur. The dense bone wall of the canal 10 provides resistance to angular or rotational stress of the prosthesis secured to the wall of the canal. Thus, the use of the instrumentation kit of the invention to compact bone clearly offers significant advantages over conventional femoral prosthesis insert preparation, wherein either nothing is done to the canal sidewalls, or wherein the canal is prepared by removing bone via drilling.

While the invention has been described in connection with what is presently considered to be the most practical and preferred embodiment, it is to be understood that the invention is not limited to the disclosed embodiment, but, on the contrary, is intended to cover various modifications and equivalent arrangements included within the scope of the appended claims.

## Claims

1. Instrumentation kit for preparing an internal wall of a cavity (10) in bone (12) for receiving a medical device within the cavity (10), the instrumentation kit comprising
a first compactor instrument (14) having a proximal portion (16) and a distal portion (18), said distal portion (18) being configured and sized relative to the dimensions of said cavity (10) so as to compact bone on said wall when the distal portion (18) of the first instrument (14) is inserted within the cavity (10), **characterised by**
at least one additional compactor instrument (30) having a proximal portion (32) and a distal portion (34), said distal portion (34) of each additional compactor (30) instrument being differently configured and sized relative to one another and being configured and sized such that after compaction of bone on the wall by at least said first instrument (14), the wall of the cavity (10) is further compacted when the distal portion (34) of each additional compactor instrument (30) is inserted within the cavity (10).

2. Instrumentation kit according to claim 1, **characterized in that** the instrumentation kit comprises at least three compactor instruments (14, 30, 50) having distal portions (18, 34, 54) of different sizes.

3. Instrumentation kit according to claim 1 or 2, **characterized in that** for preparing a femur (12) for implantation of a prosthesis therein the instrumentation kit comprises
a first compactor instrument (14) having a distal broach portion (18), said broach portion (18) being constructed and arranged to conform substantially to a proximal end (13) of the femoral canal (10) so as to compact bone in the sidewalls thereof without intentionally removing bone from the femoral canal (10),
at least one second compactor (30) instrument having a distal broach portion (34), said distal broach portion (34) being constructed and arranged to conform substantially to a shape of the prosthesis so as to further compact bone in the sidewalls of the proximal end (13) of the femoral canal (10) such that the compacted sidewalls conform substantially to the shape of the prosthesis, and
at least one third compactor instrument (50) having an elongated distal broach portion (34), said elongated broach portion (54) being constructed and arranged to extend into said femoral canal (10) and compact bone in sidewalls of said femoral canal (10) at a location distal to previously compacted sidewalls of said proximal end (13) of said femoral canal (10).

4. Instrumentation kit according to claim 3, **characterized in that** the broach portion (18) of said first compactor instrument (14) has a length (B) of approximately 70 mm, a maximum height (C) of approximately 25 mm and a width (D) in the range of approximately 2 to 4 mm.

5. Instrumentation kit according to claim 3 or 4, **characterized in that** the broach portion (34) of said second compactor instrument (30) has a maximum height (C) of approximately 25 mm and a width (D) which tapers from approximately 10 mm to approximately 5 mm towards a distal end (38) thereof.

6. Instrumentation kit according to claim 3, 4 or 5, **characterized in that** the broach portion (54) of said third compactor instrument (50) has a maximum height (C) of approximately 25 mm, a width of approximately 10 mm and a length of approximately 70 mm, and that the broach portion (54) of said third compactor instrument (50) has an elongated portion (55) with a length of approximately 90 mm, a height of approximately 9 mm and a width (D) which tapers from approximately 10 mm at one end thereof to approximately 5 mm at a distal end (56) thereof.

7. Instrumentation kit according to any one of claims 3 to 6, **characterized in that** at least one of said first compactor instrument, said second compactor instrument and said third compactor instrument comprises a plurality of instruments of different width.

8. Instrumentation kit according to any one of claims 1 to 7, **characterized in that** said proximal portions (16, 32, 52) of each of said compactor instrument (14, 30, 50) includes a head (20) formed so as to be impacted to drive the compactor instrument (14, 30, 50) into the cavity (10).

9. Instrumentation kit according to any one of claims 1 to 8, **characterized in that** each said compactor instrument (14, 30, 50) is made of stainless steel.

## Patentansprüche

1. Instrumentenausstattung zur Vorbereitung einer inneren Wand einer Knochenhöhle (10) für die Aufnahme einer medizinischen Vorrichtung innerhalb der Höhle (10), wobei die Instrumentenausstattung ein erstes Kompaktierungsinstrument (14) mit einem proximalen Teil (16) und einem distalen Teil (18) aufweist, wobei der distale Teil (18) in Bezug auf die Abmessungen der Höhle (10) derart ausgebildet und bemessen ist, dass er Knochen an der Wand kompaktiert, wenn der distale Teil (18) des ersten Instrumentes (14) in die Höhle (10) eingeführt wird, **gekennzeichnet durch** wenigstens ein weiteres Kompaktierungsinstrument (30), welches einen proximalen Teil (32) und einen distalen Teil (34) aufweist, wobei der distale Teil (34) jedes weiteren Kompaktierungsinstrumentes (30) zueinander unterschiedlich ausgebildet und bemessen ist und derart ausgebildet und bemessen ist, dass nach einer Kompaktierung von Knochen an der Wand **durch** wenigstens ein erstes Instrument (14) die Wand der Höhle (10) weiter kompaktiert wird, wenn der distale Teil (34) jedes weiteren Kompaktierungsinstrumentes (30) in die Höhle (10) eingeführt wird.

2. Instrumentenausstattung gemäß Anspruch 1, **dadurch gekennzeichnet, dass** die Instrumentenausstattung wenigstens drei Kompaktierungsinstrumente (14,30,50) aufweist, welche unterschiedlich große distale Teile (18,34,54) aufweisen.

3. Instrumentenausstattung gemäß Anspruch 1 oder 2, **dadurch gekennzeichnet, dass** die Instrumentenausstattung zur Vorbereitung des Femur (12) für die Implantation einer Prothese darin ein erstes Kompaktierungsinstrument (14) mit einem distalen Räumteil (18) aufweist, wobei der Räumteil (18) in einer der Form des proximalen Endes (13) des Femoralkanales (10) angepassten Weise derart ausgebildet und angeordnet ist, dass der Knochen an dessen Seitenwänden kompaktiert wird, ohne absichtlich Knochen von dem Femoralkanal (10) zu entfernen, dass die Instrumentenausstattung wenigstens ein zweites Kompaktierungsinstrument (30) mit einem distalen Räumteil (34) aufweist, wobei der distale Räumteil (34) in einer der Form der Prothese im Wesentlichen angepassten Form derart ausgebildet und angeordnet ist, dass er Knochen an den Seitenwänden des proximalen Endes (13) des Femoralkanals (10) weiter kompaktiert, sodass die kompaktierten Seitenwände im Wesentlichen der Form der Prothese entsprechen, und dass die Instrumentenausstattung wenigstens ein drittes Kompaktierungsinstrument (50) mit einem verlängerten distalen Räumteil (34) aufweist, wobei der verlängerte Räumteil (54) derart ausgebildet und angeordnet ist, dass er sich in den Femoralkanal (10) erstreckt und Knochen an den Seitenwänden des Femoralkanales (10) an einem relativ zu den zuvor kompaktierten Seitenwänden des proximalen Endes (13) des Femoralkanales (10) weiter distal gelegenen Ort kompaktiert.

4. Instrumentenausstattung gemäß Anspruch 3, **dadurch gekennzeichnet, dass** der Räumteil (14) des ersten Kompatkierungsinstrumentes (14) eine Länge (B) von ungefähr 70mm, eine maximale Höhe (C) von ungefähr 25mm und eine Weite (D) im Bereich von ungefähr 2 bis 4mm aufweist.

5. Instrumentenausstattung gemäß Anspruch 3 oder 4, **dadurch gekennzeichnet, dass** der Räumteil (34) des zweiten Kompaktierungsinstrumentes (30) eine maximale Höhe (C) von ungefähr 25mm und eine Weite (D) aufweist, welche sich von ungefähr 10mm auf ungefähr 5mm gegen dessen distales Ende (38) verjüngt.

6. Instrumentenausstattung gemäß Anspruch 3, 4 oder 5, **dadurch gekennzeichnet, dass** der Räumteil (54) des dritten Kompaktierungsinstrumentes (50) eine maximale Höhe (C) von ungefähr 25mm, eine Weite von ungefähr 10mm und eine Länge von ungefähr 70mm aufweist und dass der Räumteil (54) des dritten Kompaktierungsinstrumentes (50) einen verlängerten Bereich (55) mit einer Länge von ungefähr 90mm, einer Höhe von ungefähr 9mm und einer Weite (D) aufweist, welche sich von ungefähr 10mm an dessen einem Ende auf ungefähr 5mm an dessen distalem Ende (56) verjüngt.

7. Instrumentenausstattung gemäß einem der Ansprüche 3 bis 6, **dadurch gekennzeichnet, dass** wenigsten eines von dem ersten Kompatkierungsinstrument, dem zweiten Kompaktierungsinstrument und dem dritten Kompaktierungsinstrument eine Mehrzahl von Instrumenten mit unterschiedlichen Weiten umfasst.

8. Instrumentenausstattung gemäß einem der Ansprüche 1 bis 7, **dadurch gekennzeichnet, dass** die proximalen Teile (16,32,52) jedes der Kompaktierungsinstrumente (14,30,50) einen Kopf (20) umfasst, sodass durch Druckausübung das Kompaktierungsinstrument (14,30,50) in die Höhle (10) eingetrieben wird.

9. Instrumentenausstattung gemäß einem der Ansprüche 1 bis 8, **dadurch gekennzeichnet, dass** jedes Kompaktierungsinstrument (14,30,50) aus rostfreiem Stahl hergestellt ist.

## Revendications

1. Lot d'instruments pour préparer une paroi interne d'une cavité (10) dans l'os (12) en vue de la pose d'un dispositif médical à l'intérieur de la cavité (10), le lot d'instruments comprenant :
un premier instrument de compactage (14) ayant une partie proximale (16) et une partie distale (18), ladite partie distale (18) étant configurée et dimensionnée relativement aux dimensions de ladite cavité (10) de manière à compacter l'os sur ladite paroi lorsque la partie distale (18) du premier instrument de compactage (14) est insérée à l'intérieur de la cavité (10), **caractérisé par** :
au moins un instrument de compactage supplémentaire (30) ayant une partie proximale (32) et une partie distale (34), ladite partie distale (34) de chaque instrument de compactage supplémentaire (30) étant distinctement configurée et dimensionnée relativement l'une à l'autre, et l'instrument étant configuré et dimensionné de sorte qu'après le compactage de l'os sur la paroi par au moins ledit premier instrument de compactage (14), la paroi de la cavité (10) est davantage compactée lorsque la partie distale (34) de chaque instrument de compactage supplémentaire (30) est insérée à l'intérieur de la cavité (10).

2. Lot d'instruments selon la revendication 1, **caractérisé en ce que** le lot d'instruments comprend au moins trois instruments de compactage (14, 30, 50) ayant des parties distales (18, 34, 54) de différentes tailles.

3. Lot d'instruments selon la revendication 1 ou 2, **caractérisé en ce que** pour préparer un fémur (12) à la pose d'une prothèse à l'intérieur de celui-ci, le lot d'instruments comprend :
un premier instrument de compactage (14) ayant une partie distale de broche (18), ladite partie de broche (18) étant conçue et agencée pour s'adapter substantiellement à une extrémité proximale (13) du canal fémoral (10) de manière à compacter l'os dans les parois latérales de celui-ci sans intentionnellement retirer de l'os du canal fémoral (10),
au moins un second instrument de compactage (30) ayant une partie distale de broche (34), ladite partie distale de broche (34) étant conçue et agencée pour s'adapter substantiellement à une forme de la prothèse de manière à davantage compacter l'os dans les parois latérales de l'extrémité proximale (13) du canal fémoral (10) de sorte que les parois latérales compactées s'adaptent substantiellement à la forme de la prothèse, et
au moins un troisième instrument de compactage (50) ayant une partie distale de broche allongée (34), ladite partie distale de broche allongée (34) étant conçue et agencée pour s'étendre dans ledit canal fémoral (10) et à compacter l'os dans les parois latérales dudit canal fémoral (10) à un emplacement distal aux parois latérales précédemment compactées de ladite extrémité proximale (13) dudit canal fémoral (10).

4. Lot d'instruments selon la revendication 3, **caractérisé en ce que** la partie de broche (18) dudit premier instrument de compactage (14) a une longueur (B) d'approximativement 70 mm, une hauteur maximale (C) d'approximativement 25 mm et une largeur (D) dans la gamme d'approximativement 2 à 4 mm.

5. Lot d'instruments selon la revendication 3 ou 4, **caractérisé en ce que** la partie de broche (34) dudit second instrument de compactage (30) a une hauteur maximale (C) d'approximativement 25 mm et une largeur (D) ayant une forme conique d'approximativement 10 mm à approximativement 5 mm dans la direction d'une extrémité distale (38) de celle-ci.

6. Lot d'instruments selon la revendication 3, 4 ou 5, **caractérisé en ce que** la partie de broche (54) dudit troisième instrument de compactage (50) a une hauteur maximale (C) d'approximativement 25 mm, une largeur d'approximativement 10 mm et une longueur d'approximativement 70 mm, et **en ce que** la partie de broche (54) dudit troisième instrument de compactage (50) a une partie allongée (55) ayant une longueur d'approximativement 90 mm, une hauteur d'approximativement 9 mm et une largueur (D) ayant une forme conique d'approximativement 10 mm à une extrémité de celle-ci à approximativement 5 mm à une extrémité distale (56) de celle-ci.

7. Lot d'instruments selon l'une quelconque des revendications 3 à 6, **caractérisé en ce qu'**au moins un dudit premier instrument de compactage, dudit second instrument de compactage et dudit troisième instrument de compactage, comprend une pluralité d'instruments de largeurs différentes.

8. Lot d'instruments selon l'une quelconque des revendications 1 à 7, **caractérisé en ce que** lesdites parties proximales (16, 32, 52) de chacun desdits instruments de compactage (14, 30, 50) comporte une tête (20) formée en vue d'être enclavée afin de diriger les instruments de compactage (14, 30, 50) dans la cavité (10).

9. Lot d'instruments selon l'une quelconque des revendications 1 à 8, **caractérisé en ce que** chacun des instruments de compactage (14, 30, 50) est réalisé en acier inoxydable.
